# EUROPEAN PATENT SPECIFICATION

(11) **EP 1 624 813 B1**
(45) Date of publication and mention of the grant of the patent: **02.11.2006**
(21) Application number: 04733365.3
(22) Date of filing: 17.05.2004
(51) Int. Cl.: A61B 17/16

(54) **A CUTTING TOOL FOR USE IN ORTHOPAEDIC SURGERY**
ORTHOPÄDISCHES SCHNEIDEINSTRUMENT
OUTIL COUPANT POUR CHIRURGIE ORTHOPEDIQUE

(30) Priority: 17.05.2003 GB 0311374
(43) Date of publication of application: 15.02.2006
(73) Proprietor: Depuy International Limited, Beeston, Leeds LS11 8DT (GB)
(72) Inventor: DOWER, Liam, Huddersfield UDX 8JX (GB)
(74) Representative: Belcher, Simon James
(86) International application number: PCT/GB2004/002115
(87) International publication number: WO 2004/100804

(56) References cited:
- WO-A-02/49516
- US-A- 2 785 673
- US-B1- 6 245 074

## Description

This invention relates to a cutting tool for preparing a cavity in a bone for receiving a component of an orthopaedic joint prosthesis.

A rotationally symmetrical cavity in a bone can be prepared to receive a component of an orthopaedic joint prosthesis using a cutting tool which is rotationally symmetrical. The cutting tool will usually be an approximately hemispherical shell which is hollow. It will include a formation in its interior so that it can be engaged by an instrument by which rotational movement can be imparted to the shell. The formation can include for example a bar which extends across the shell. The axis of rotation of the shell will usually be coincident with the polar axis of the shell. The peripheral edge of the shell will usually define a plane which is perpendicular to the polar axis. The angle subtended at the centre of the sphere, of which the surface of the shell forms a part, between the peripheral edge at two diametrically opposite points will generally be close to 180°, for example at least about 170°. The cutting tool will have cutting teeth on its external spherical surface so that it cuts the bone to form the cavity by rotation when in contact with the bone. The cutting teeth can be in the form of elongate slits, or generally round perforations. Such cutting tools are known, for example as disclosed in US-5203653 and US-5709688. Preferably, the arrangement of the cutting teeth on the surface of the tool is such that the axis around which torque, arising from the accumulated resistance to rotation of the tool when rotated against bone tissue, is applied to the tool is approximately coincident with the polar axis of the tool.

Cutting tools of this kind are used after preparatory steps which include forming an incision and removal or displacement of any obstructing tissue. A tool is mounted on a suitable drive instrument which can be used to impart rotational movement to the tool and inserted through the incision to engage the relevant surface of the bone.

It can be desirable to minimise the size of an incision through which access is gained to a patient's bone to perform surgery on it, especially in order to reduce the period taken for the patient to recover from the surgery. The size of a cutting tool, when mounted on the drive instrument, can determine the minimum size of the incision in a procedure. It can therefore be desirable to reduce the size of a shell cutting tool, while still ensuring that the tool can cut bone to form a cavity of desired shape and size.

US 2,785,673 discloses a hemispherical shell cutting tool according to the preamble of claim 1.

The present invention provides a hemispherical shell cutting tool, in which the shell has at least three portions cut out from it such that the peripheral edge of the shell is intact at two diametrically opposite points, and the centre of mass of the shell lies on the polar axis.

Accordingly, in one aspect, the invention provides a cutting tool for preparing a cavity in a bone for receiving a component of an orthopaedic joint prosthesis, in which the bone contacting surface of the component is approximately hemispherical, in which the shape of the tool is based on a hemispherical shell, in which the shell has at least three portions cut out from it extending from the peripheral edge of the shell part way towards its pole, in which the cut out portions are such that (a) the peripheral edge of the shell is intact at two diametrically opposite points, and (b) the centre of mass of the shell lies on the polar axis.

The tool of the invention has the advantage that its effective width is reduced compared with a full hemispherical shell by virtue of the cutout portions. However, the size of the recess which can be formed in a bone using the cutting tool is determined by the shape of the tool in the regions in which the peripheral edge is intact: this can be the same as in a conventional hemispherical shell in which there are no cutout portions. Accordingly, it is possible by virtue of the tool of the invention for the tool to be inserted through an incision that is smaller than is necessary with a conventional full hemispherical shell, and to obtain a recess which has the same shape as that which is obtained with the conventional shell.

The cutout portions that are features of the tool of the invention mean that there can be fewer cutting teeth by which to form the cavity in the bone. However, it has been found according to the present invention that adequate cutting is possible with the tool of the invention, notwithstanding the loss of some of its external surface on which cutting teeth can be arranged.

It is an advantage of the present invention that, by arranging the cut out portions such that the centre of mass of the shell lies on the polar axis, the tool can be rotated during the cutting operation without the generation of unacceptable transverse vibration movement.

It is a further advantage of the present invention that the full width of the shell is preserved at two diametrically opposed points. This allows the shell to include a bar extending across the interior of the shell, from one side to the opposite other side, by which the shell can be engaged by an instrument for manipulation. The bar can be provided at the open face of the shell. The bar can be recessed within the shell, at a point between the open face and the interior surface at the pole. The use of a bar to provide a connection between a cutting tool shell and a rotational drive instrument is well established and features which are known in this context for forming such a connection can be used in the present invention.

The cutting tool of the invention can have at least three cut out portions, or at least four cut out portions, or more. The number of cut out portions will be selected according to the size of the incision through which the tool is to be inserted to engage the patient's bone, and to the cutting area which is to be retained on the external surface of the shell. It is an advantage to include three cut out portions because this can facilitate manipulation of the tool where space is restricted, especially to get the tool past soft tissue which partially obstructs the bone on which the procedure is to be performed. It will often be preferred for the tool of the invention to include three cut out portions.

Preferably, the peripheral edge of the shell is intact around at least about 30% of the length of the circumference of the shell at the edge, more preferably at least about 35%, especially at least about 40%. Preferably, the peripheral edge of the shell is intact around not more than about 80% of the length of the circumference of the shell at the edge, more preferably not more than about 70%, especially not more than about 60%.

Preferably, at least one of the cut out portions extends over at least about 15% of the length of the circumference of the shell at the peripheral edge, more preferably at least about 20%, for example at least about 25%. This can enable appreciable reductions in the transverse dimension of the tool to be achieved.

Preferably, the edges of the cut out portions where the cut out portions meet the intact peripheral edge of the shell extend radially towards the pole of the shell. However, the cut out portions can have different shapes. For example, each cut out portion can be defined by a chord which extends directly from one edge of that cut out portion, where it meets the intact peripheral edge of the shell, to its opposite edge. Other shapes of cut out portion are also envisaged. For example, a curved edge can extend from one side of a cut out portion, where it meets the intact peripheral edge of the shell, to its opposite side.

Preferably, the size of one of the cut out portions differs from the size of another of the cut out portions. It will often be the case that, in order to achieve balance of the tool with the centre of mass on the polar axis, the size of two of the cut out portions will be approximately the same and different from the size of the or each other cut out portions.

Preferably, the size of one of the portions of the shell in which the peripheral edge is intact differs from the size of another of the said portions. It will often be the case that, in order to achieve balance of the tool with the centre of mass on the polar axis, the size of two of the intact portions will be approximately the same and different from the size of the or each other intact portions.

The angle subtended at the centre of the sphere, of which the surface of the shell forms a part, between the peripheral edge at the said diametrically opposite points will generally be less than 180° so that the tool does not generate a cavity whose diameter at the open face is less than the maximum diameter of the tool. Preferably, the said angle is at least about 150°, preferably at least about 170°.

Preferably, the shell is intact in a circular region around the pole. This has the advantage of optimising the area of the external surface of the shell in the region in which maximum axial force will have to be withstood. This has the advantage of optimising the surface that is available for providing cutting teeth and also optimising the strength of the tool so that to enable it to withstand applied axial forces. Preferably, the ratio of the distance from the edge of the circular region to the pole to the ratio of the distance of the peripheral edge of the shell to the pole (both distances being measured along the spherical surface of the shell) is not more than about 0.5, more preferably not more than about 0.4, for example about 0.3. Preferably, the ratio of the distance from the edge of the circular region to the pole to the ratio of the distance of the peripheral edge of the shell to the pole (both distances being measured along the spherical surface of the shell) is not less than about 0.1, more preferably not less than about 0.2.

The tool of the invention will generally have at least one cutter tooth which is protrudes outwardly from the spherical surface of the shell. This can be formed by cutting the material of the shell and deforming the material outwardly. The exposed edge of the cut material should be sharp in order to be able to cut the bone tissue, either as a result of the cutting step or as a result of a subsequent sharpening step. The cutter tooth can be in the form of an elongate slit. Preferably, the tool of the invention has cutter teeth in the form of generally round perforations, especially a plurality of the said teeth arranged over the external surface of the shell. Each such cutter tooth is preferably formed by cutting the material of the shell part way around the edge of a generally round opening, and deforming the material of the shell by bending it at that part of the edge of the opening where the material is not cut. The transverse dimension of such an opening (which will be a diameter when the opening is circular) is preferably at least about 1.5 mm, more preferably at least about 2.0 mm, especially at least about 2.5 mm.

Preferably, cutting teeth are distributed approximately evenly over the surface of the shell so that the number of teeth per unit area of the external surface thereof is approximately constant over the entire surface. It will be appreciated however that this can only be approximated because of the relative sizes of the teeth and the surface of the shell.

It is an advantage to use a plurality of circular openings for the cutter teeth because it allows the distribution of cutter teeth over the surface of the tool to be varied. For example, it can be preferred to have a greater concentration of the cutter teeth close to the pole of the tool, and compared with close to the peripheral edge of the tool.

The tool of the invention can be made from materials which are suitable for surgical cutting tools. Particularly preferred materials include certain stainless steels. The tool can be formed by casting. When the tool includes a bar which extends across the interior of the shell, this can be formed integrally with the shell in a casting operation, or separately and then fastened to the shell, for example by welding. Cutter teeth can be formed on the shell in a casting step, or separate from the step of forming the shell, for example in a subsequent operation involving for example cutting or machining or both.

Embodiments of the invention will now be described by way of example with reference to the accompanying drawings, in which:
Figure 1 is an isometric view from below of a reamer according to the invention.
Figure 2 is a schematic plan view of the reamer which is shown in Figure 1.
Figure 3 is a schematic plan view of an alternative embodiment of reamer.
Figures 4a and 4b illustrate how the a reamer according to the invention can be designed.
Figure 5 is a schematic plan view of a further embodiment of reamer.

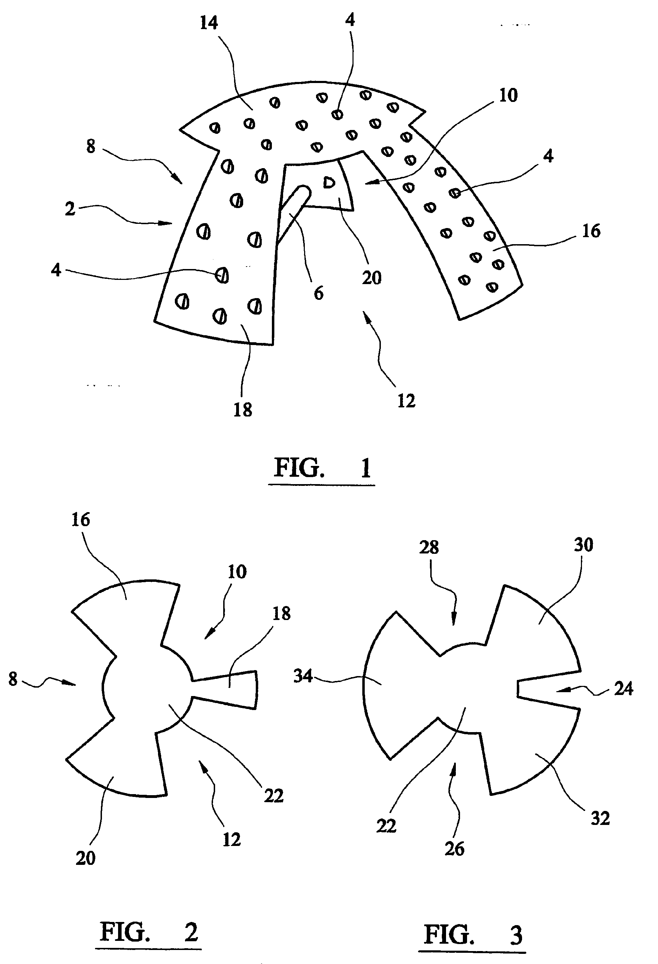

Referring to the drawings, Figure 1 shows a reamer 2 which comprises a hollow shell formed from an inert metal such as a stainless steel. The material of the shell is about 0.5 mm thick. The shell has cutter teeth 4 which extend outwardly from its external surface, each being formed by cutting perforations into the shell and deforming the material of the shell outwardly.

The shell has a bar 6 extending across it from one side to a diametrically opposite point on the other side, by which the shell can be fastened to a drive instrument which can be used to impart rotation drive to the shell. Clamps by which the bar can be engaged by the instrument are known in this context.

The external surface of the shell forms part of a hemisphere. The angle subtended at the centre of the sphere (of which the surface of the shell forms a part) between the peripheral edge at the opposite ends of the bar 6 is 175°. The shell has portions 8, 10, 12 cut from it which extend from the peripheral edge of the shell part way towards the pole. The cut out portions are such that the peripheral edge of the shell is intact at the diametrically opposite points where the bar 6 is fastened to the shell. The cut out portions are such that the centre of mass of the shell lies on the polar axis.

The external surface of the shell includes an intact region 14 around the pole which is circular when the component is viewed in plan, and whose surface forms part of a sphere.

The location of the centre of mass on the pole of the shell is achieved by suitable arrangement of the cut out portions. This is illustrated in Figure 2 for the tool shown in Figure 1. The following table sets out the angles subtended at the pole by the cut out portions 8,10, 12, and by the portions 16, 18, 20 of the shell which remain between the cut out portions:

| | Portion | Angle (°) |
|---|---|---|
| Cut out portions | 8 | 90 |
| | 10 | 60 |
| | 12 | 60 |
| Portions with cutters | 16 | 60 |
| | 18 | 30 |
| | 20 | 60 |

The embodiment shown in Figures 1 and 2 includes a portion 22 around the pole whose shape is that of part of a sphere. Each of the cut out portions 8, 10, 12 is defined by edges which extend radially from the circular portion 22 to the peripheral edge of the shell, and by the edge of the part spherical portion 22.

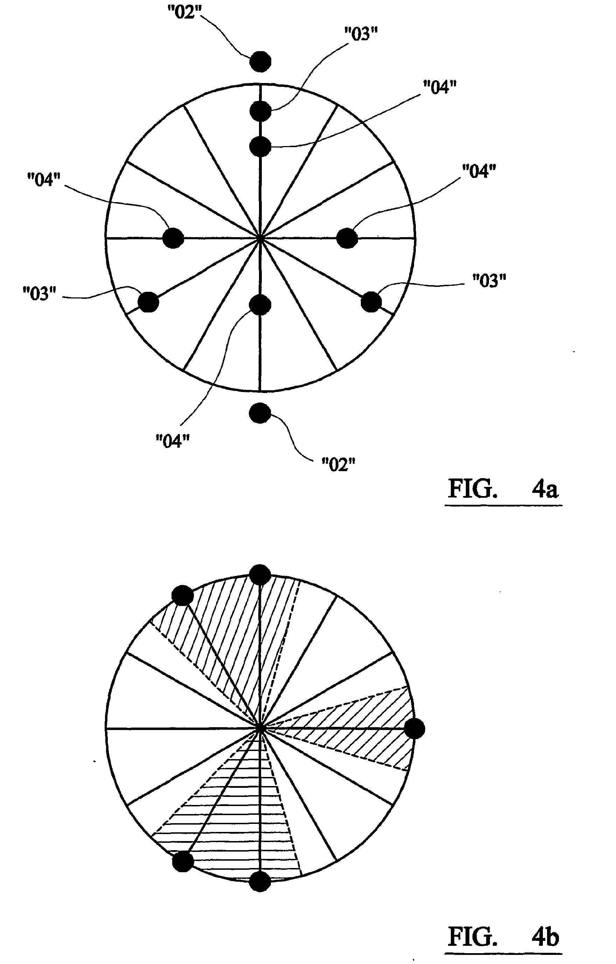

The embodiment which is shown in Figure 3 differs from that shown in Figures 1 and 2 in that the angular arrangement of the cut out portions 24, 26, 28, and the portions of the shell 30, 32, 34, which remain between the cut out portions are reversed.

In the embodiment shown in Figure 3, each of the cut out portions 36, 38, 40 is defined by an edge which extends from one side of that cut out portion, where it meets the intact peripheral edge of the shell, to its opposite side. The edge can extend straight between the two sides (when the shell is viewed along a radius) or it can be curved.

Figures 4a and 4b illustrate the principle which can be used to design an appropriate arrangement of cut out portions on a hemispherical shell to embody the present invention. The distribution of the cut out portions can be modelled on the arrangement of holders in a centrifuge for fluid containers. In order for the centrifuge to remain balanced when it spins, it is important for the centre of mass of the loaded holders to lie on the axis of rotation.

Figure 4a shows arrangements of holders for 2, 3 and 4 fluid samples respectively in a centrifuge which has twelve radially spaced holder arms. The respective holders are labelled "02", "03" and "04".

A balanced arrangement can be created for numbers of samples other than 2, 3 and 4 by combining the arrangements for the smaller numbers. Figure 4b shows the arrangement of holders for 5 fluid samples, and it on this arrangement that the arrangement of cut out portions in the embodiment of Figures 1 and 2 is based.

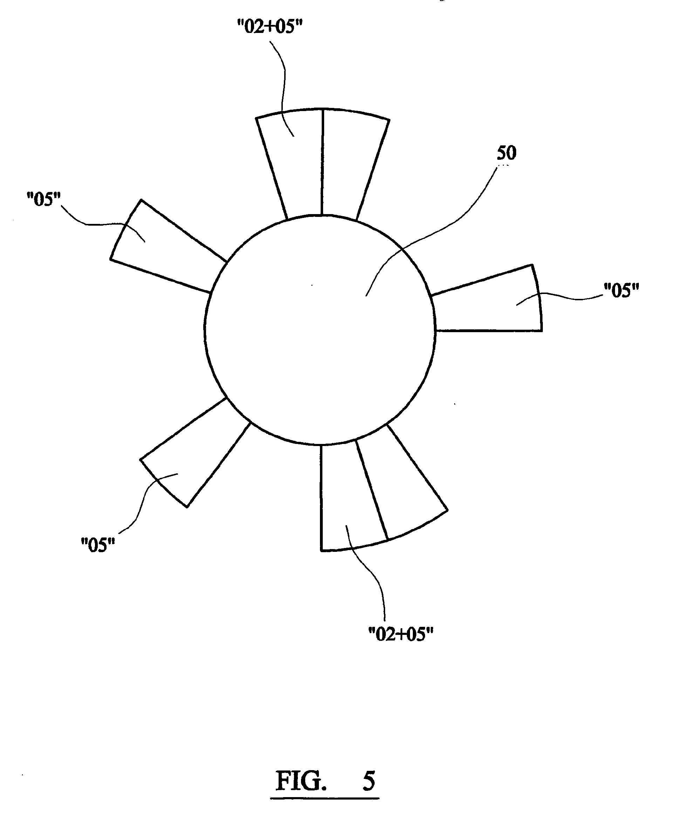

Figure 5 shows a further embodiment of reamer in which the distribution of the cut out portions is modelled on the arrangement of holders in a centrifuge for fluid containers, in which there are 20 such holders. A pattern of cutting portions for the reamer is shown which is based on a combination of 5 rotationally balanced fluid samples and 2 rotationally balanced fluid samples. Accordingly, three of the arms, labelled "05", which extend from the central partial spherical section 50 are relatively narrow, and two of the arms, labelled "02+05" are relatively wide.

It will be understood that, of the three embodiments shown in Figures 2, 3 and 5, the Figure 2 embodiment is preferred from the particular point of view of reducing the radial extent of the reamer to enable insertion through a small incision.

## Claims

1. A cutting tool (2) for preparing a cavity in a bone for receiving a component of an orthopaedic joint prosthesis, in which the bone contacting surface of the component is approximately hemispherical, in which the shape of the tool is based on a hemispherical shell, **characterised in that** the shell has at least three portions (8, 10, 12) cut out from it extending from the peripheral edge of the shell part way towards its pole, in which the cut out portions are such that (a) the peripheral edge of the shell is intact at two diametrically opposite points, and (b) the centre of mass of the shell lies on the polar axis.

2. A cutting tool (2) as claimed in claim 1, in which the peripheral edge of the shell is intact around at least about 30% of the length of the circumference of the shell at the edge.

3. A cutting tool (2) as claimed in claim 1, in which the peripheral edge of the shell is intact around not more than about 80% of the length of the circumference of the shell at the edge.

4. A cutting tool (2) as claimed in claim 1, in which at least one of the cut out portions (8, 10, 12) extends over at least about 15% of the length of the circumference of the shell at the peripheral edge.

5. A cutting tool (2) as claimed in claim 1, in which the angle subtended at the centre of the sphere, of which the surface of the shell forms a part, between the peripheral edge at the said diametrically opposite points is at least about 150°, preferably at least about 170°.

6. A cutting tool (2) as claimed in claim 1, in which the size of one of the cut out portions (8, 10, 12) differs from the size of another of the cut out portions.

7. A cutting tool (2) as claimed in claim 1, in which the shell is intact in a region around the pole (22).

8. A cutting tool (2) as claimed in claim 7, in which the shape of the external surface of the shell in the region around the pole (22) is generally that of a part of a sphere.

9. A cutting tool (22) as claimed in claim 7, in which the ratio of the distance from the edge of the circular region (22) to the pole to the ratio of the distance of the peripheral edge of the shell to the pole (both distances being measured along the spherical surface of the shell) is not more than about 0.5.

10. A cutting tool (2) as claimed in claim 7, in which the ratio of the distance from the edge of the circular region (22) to the pole to the ratio of the distance of the peripheral edge of the shell to the pole (both distances being measured along the spherical surface of the shell) is not less than about 0.1.

11. A cutting tool (2) as claimed in claim 1, which has at least one cutter tooth (4) which is protrudes outwardly from the spherical surface of the shell.

12. A cutting tool (2) as claimed in claim 1, which includes a bar (6) which extends across the interior of the shell, from one side to the opposite other side, by which the shell can be engaged by an instrument for manipulation.

## Patentansprüche

1. Schneidwerkzeug (2) zum Herstellen einer Kavität in einem Knochen zur Aufnahme einer Komponente einer orthopädischen Gelenkprothese, bei welcher die mit dem Knochen in Kontakt kommende Oberfläche der Komponente annähernd halbkugelförmig ist, wobei die Form des Werkzeuges auf einer halbkugelförmigen Schale basiert, **dadurch gekennzeichnet, daß** die Schale wenigstens drei aus ihr ausgeschnittene Abschnitte (8, 10, 12) aufweist, welche sich vom Umfangsrand der Schale über einen Teil des Weges zu ihrem Pol erstrecken, wobei die ausgeschnittenen Abschnitte so ausgebildet sind, daß (a) der Umfangsrand der Schale an zwei einander gegenüberliegenden Punkten erhalten ist, und (b) der Massenschwerpunkt der Schale auf der polaren Achse liegt.

2. Schneidwerkzeug (2) nach Anspruch 1, bei dem der Umfangsrand der Schale über wenigstens etwa 30% der Länge des Schalenumfangs an dem Rand erhalten ist.

3. Schneidwerkzeug (2) nach Anspruch 1, bei dem der Umfangsrand der Schale über nicht mehr als etwa 80% der Länge des Schalenumfangs an dem Rand erhalten ist.

4. Schneidwerkzeug (2) nach Anspruch 1, bei dem sich wenigstens einer der ausgeschnittenen Abschnitte (8, 10, 12) über wenigstens etwa 15% der Länge des Schalenumfangs an dem Umfangsrand erstreckt.

5. Schneidwerkzeug (2) nach Anspruch 1, bei dem der Winkel am Zentrum der Kugelfläche, von welcher die Oberfläche der Schale einen Teil bildet, zwischen den einander diametral gegenüberliegenden Punkten am Umfangsrand wenigstens etwa 150° beträgt, vorzugsweise wenigstens etwa 170°.

6. Schneidwerkzeug (2) nach Anspruch 1, bei dem die Größe eines der ausgeschnittenen Abschnitte (8, 10, 12) unterschiedlich zu der Größe eines anderen ausgeschnittenen Abschnittes ist.

7. Schneidwerkzeug (2) nach Anspruch 1, bei dem die Schale in einem Bereich um den Pol (22) herum erhalten ist.

8. Schneidwerkzeug (2) nach Anspruch 7, bei dem die Form der äußeren Fläche der Schale im Bereich um den Pol (22) herum im wesentlichen diejenige eines Teils einer Kugelfläche ist.

9. Schneidwerkzeug (2) nach Anspruch 7, bei dem das Verhältnis des Abstandes von dem Rand des kreisförmigen Bereiches (22) zum Pol zu dem Verhältnis des Abstandes des Umfangsrandes zum Pol (beide Abstände entlang der kugelförmigen Oberfläche der Schale gemessen) nicht mehr als etwa 0,5 beträgt.

10. Schneidwerkzeug (2) nach Anspruch 7, bei dem das Verhältnis des Abstandes von dem Rand des kreisförmigen Bereiches (22) zum Pol zu dem Verhältnis des Abstandes des Umfangsrandes zum Pol (beide Abstände entlang der kugelförmigen Oberfläche der Schale gemessen) nicht weniger als etwa 0,1 beträgt.

11. Schneidwerkzeug (2) nach Anspruch 1, welches wenigstens einen Schneidzahn (4) aufweist, welcher von der sphärischen Oberfläche der Schale vorsteht.

12. Schneidwerkzeug (2) nach Anspruch 1, welches einen Stab (6) od.dgl. aufweist, der sich von einer Seite zur gegenüberliegenden anderen Seite über das Innere der Schale erstreckt, durch welchen die Schale zwecks Betätigung mit einem Instrument in Eingriff gebracht werden kann.

## Revendications

1. Outil coupant (2) pour préparer une cavité dans un os pour recevoir un élément d'une prothèse d'articulation orthopédique, dans lequel la surface au contact de l'os de l'élément est approximativement hémisphérique, dans lequel la forme de l'outil est basée sur une carcasse hémisphérique, **caractérisé en ce que** la carcasse a au moins trois parties (8, 10, 12) découpées en lui s'étendant du bord périphérique de la carcasse en partie vers son pôle, dans lequel les parties découpées sont telles que (a) le bord périphérique de la carcasse est intact aux deux points diamétralement opposés et (b) le centre de la masse de la carcasse reste sur l'axe polaire.

2. Outil coupant (2) comme revendiqué dans la revendication 1, dans lequel le bord périphérique de la carcasse est intact autour d'au moins environ 30 % de la longueur de la circonférence de la carcasse vers le bord.

3. Outil coupant (2) comme revendiqué dans la revendication 1, dans lequel le bord périphérique de la carcasse est intact autour de pas plus qu'environ 80 % de la longueur de la circonférence de la carcasse vers le bord.

4. Outil coupant (2) comme revendiqué dans la revendication 1, dans lequel au moins l'une des parties découpées (8, 10, 12) s'étend sur au moins environ 15 % de la longueur de la circonférence de la carcasse vers le bord périphérique.

5. Outil coupant (2) comme revendiqué dans la revendication 1, dans lequel l'angle sous-tendu au centre de la sphère, dont la surface de la carcasse forme une partie, entre le bord périphérique vers lesdits points diamétralement opposés fait au moins environ 150°, de préférence, au moins environ 170°.

6. Outil coupant (2) comme revendiqué dans la revendication 1, dans lequel la taille de l'une des parties découpées (8, 10, 12) est différente de la taille d'une autre des parties découpées.

7. Outil coupant (2) comme revendiqué dans la revendication 1, dans lequel la carcasse est intacte dans une région autour du pôle (22).

8. Outil coupant (2) comme revendiqué dans la revendication 7, dans lequel la forme de la surface externe de la carcasse dans la région autour du pôle (22) est généralement celle d'une partie d'une sphère.

9. Outil coupant (2) comme revendiqué dans la revendication 7, dans lequel le rapport de la distance depuis le bord de la région circulaire (22) jusqu'au pôle sur le rapport de la distance du bord périphérique de la carcasse jusqu'au pôle (les deux distances étant mesurées le long de la surface sphérique de la carcasse) ne fait pas plus qu'environ 0,5.

10. Outil coupant (2) comme revendiqué dans la revendication 7, dans lequel le rapport de la distance depuis le bord de la région circulaire (22) jusqu'au pôle sur le rapport de la distance du bord périphérique de la carcasse jusqu'au pôle (les deux distances étant mesurées le long de la surface sphérique de la carcasse) n'est pas inférieur à environ 0,1.

11. Outil coupant (2) comme revendiqué dans la revendication 1, qui a au moins une dent de coupe (4) qui fait saillie vers l'extérieur depuis la surface sphérique de la carcasse.

12. Outil coupant (2) comme revendiqué dans la revendication 1, qui inclut une barre (6) qui s'étend d'un côté à l'autre de l'intérieur de la carcasse, depuis un côté jusqu'à l'autre côté opposé, au moyen de laquelle la carcasse peut être mise en prise par un instrument pour une manipulation.
